# EUROPEAN PATENT SPECIFICATION

(11) **EP 3 157 582 B1**
(45) Date of publication and mention of the grant of the patent: **15.02.2023**
(21) Application number: 15736008.2
(22) Date of filing: 18.06.2015
(51) Int. Cl.: A61L 2/28, G02B 5/124, B29D 11/00

(54) **PROTECTION OF CONTACT LENSES FROM MICROBIAL CONTAMINATION CAUSED BY HANDLING**
SCHUTZ VON KONTAKTLINSEN VOR MIKROBIELLER, DURCH HANDHABUNG VERURSACHTER KONTAMINATION
PROTECTION DE LENTILLES DE CONTACT CONTRE UNE CONTAMINATION MICROBIENNE PROVOQUÉE PAR LA MANIPULATION

(30) Priority: 19.06.2014 US 201462014348 P
(43) Date of publication of application: 26.04.2017
(73) Proprietor: CooperVision International Limited, Fareham PO15 5RL (GB)
(72) Inventor: MALTSEVA, Inna, Pleasanton, California 94588 (US); MORRIS, Carol Ann, Pleasanton, California 94588 (US); KHONG, Kathleen, Pleasanton, California 94588 (US); LUK, Andrew, Pleasanton, California 94588 (US)
(74) Representative: Abel & Imray LLP
(86) International application number: PCT/GB2015/051789
(87) International publication number: WO 2015/193676

(56) References cited:
- EP-A1- 1 473 584
- EP-A1- 2 666 484
- WO-A1-2014/091574
- WO-A1-2014/096852
- WO-A2-2011/005937
- WO-A2-2014/096854
- DE-U1- 29 916 848
- FR-A1- 2 542 463
- JP-A- 2001 228 444
- US-A1- 2010 104 528
- US-B1- 8 349 303
- Anonymous: "Polylysine - Wikipedia, the free encyclopedia", , 29 November 2013 (2013-11-29), XP055214002, Retrieved from the Internet: URL:https://en.wikipedia.org/w/index.php?t itle=Polylysine&oldid=583780775 [retrieved on 2015-09-17]
- Luensmann Doerte ET AL: "The efficiency of contact lens care regimens on protein removal from hydrogel and silicone hydrogel lenses", Molecular vision, 20 January 2010 (2010-01-20), pages 79-92, XP055907474, United States Retrieved from the Internet: URL:https://www.ncbi.nlm.nih.gov/pmc/artic les/PMC2808856/pdf/mv-v16-79.pdf [retrieved on 2022-03-31]

## Description

### FIELD

The field of the invention is contact lenses packages.

### BACKGROUND

Microbial contamination of contact lenses is a serious public health concern due to its implications in ocular infiltrates, infections and microbial keratitis. Studies have demonstrated that lens handling greatly increases the incidence of microbial contamination of lenses (see e.g. Szczotka-Flynn et al., Eye Contact Lens (2010) 36(2): 116-29). Daily disposable contact lens wearers can be particularly at risk for contamination due to the non-compliant storage of contact lenses after use in their original packaging solution and subsequent reuse (Boost et al., Optom Vis Sci (2011) 88(12:1409-13).

ε-polylysine is a homopolymer of about 25-35 residues of L-lysine in which the epsilon-amino and carboxyl groups of L-Lysine are linked. It is a naturally-occurring polymer produced by *Streptomyces* species. It has broad-spectrum antimicrobial activity and has been widely used as a food preservative in Japan and as an additive in a variety of consumer products. The use of ε-polylysine in contact lens care solutions has been described (see e.g. U.S. Pat. No. 6,187,264, and U.S. Pat. Publ. No. 2005/0074467). Antimicrobial hydrogels made from epsilon-poly-L-lysine-graft-methacrylamide have been reported (Zhou et al., Biomaterials 32 (2011) 2704-2712).

Other background publications include co-pending U.S. Pat. Appl. No. 14/109,976, U.S. Pat. Publ. No. 2012/0074352, U.S. Pat. Publ. No. 2011/0071091, U.S. Pat. Publ. No. 2005/0074467, U.S. Pat. Publ. No. 2004/0135967, U.S. Pat. No. 4,168,112, U.S. Pat. No. 7,282,214, U.S. Pat. No. 7,402,318, EP Pat. No. 1328303B1, and PCT Publ. No. WO94/13774. WO 2011/005937 A2 describes siloxane monomers useful in the manufacture of ophthalmic devices which may be packaged in solutions containing polypeptides such as poly(lysine). EP 1 473 584 discloses a contact lens solution for washing, disinfecting and preserving containing polylysine, together with polyphosphoric acid and/or its salt to prevent polylysine adsorption. EP2666484 discloses a liquid formulation comprising polylysine polypeptide as anti-microbial agent.

### SUMMARY

The invention provides a sealed, autoclaved contact lens package comprising a sterile, unworn contact lens and a contact lens packaging solution, said contact lens packaging solution comprising epsilon polylysine (εPLL), wherein the unworn contact lens exhibits reduced contamination from at least one microbe, wherein said reduced contamination is determined by a test where *Pseudomonas aeruginosa* (PA) introduced to the lens during removal from the contact lens packaging solution compared to a control contact lens packaged in an identical contact lens packaging solution but without said εPLL, the unworn contact less resulting in less than a two log kill of PA compared to the control contact lens when tested after 24 hours incubation with 104 CFU PA using an in vitro bioactivity assay, wherein the contact lens package comprises (a) a plastic base member comprising a cavity which retains the contact lens and the packaging solution and (b) a removable foil attached to the flange region to provide the sealed contact lens package.

### DETAILED DESCRIPTION

We demonstrate that the initial removal of contact lenses from their original sterile packaging can result in significant microbial contamination of the lenses, even when handled immediately after hand washing. We have found that the addition of epsilon polylysine (εPLL) to the contact lens packaging solution can reduce or completely eliminate such lens-handling contamination. This protective effect does not require that the εPLL be attached to the contact lens material. Thus, the present invention is advantageous in that it is applicable to any type of contact lens material. It is particularly advantageous in situations where prolonged contact of a wearer's eye with an antimicrobial agent is not desired or is unnecessary. Disclosed herein is a sealed contact lens package comprising a sterile, unworn contact lens and a contact lens packaging solution comprising epsilon polylysine (εPLL) in an amount effective to reduce or completely eliminate microbial contamination introduced to the lens during its removal from the package. As used herein, "unworn" means that the contact lens has never been placed on an eye, and "sealed" means having a water-tight seal.

With the present invention, a sealed contact lens package comprising a sterile, unworn contact lens and a contact lens packaging solution comprising epsilon polylysine (εPLL) according to claim 1 is provided. The unworn contact lens exhibits reduced contamination from at least one microbe compared to a control contact lens packaged in an identical contact lens packaging solution but without said εPLL. The reduced contamination can be determined by a test where *Pseudomonas aeruginosa* (PA), introduced to the lens during removal from the contact lens packaging solution compared to a control contact lens packaged in an identical contact lens packaging solution but without said εPLL. The at least one microbe can comprise, consists of, consists essentially of, or include *Pseudomonas aeruginosa* and/or *Staphylococcus aureus,* and/or the at least one microbe is a microbe(s) found in microbial keratitis and/or at least one microbe causing at least in part or entirely ocular infiltrates and/or infections. The reduced contamination is determined in the test in which the unworn contact less results in less than a two log kill of PA compared to the control contact lens when tested after 24 hours incubation with 104 CFU PA using an in vitro bioactivity assay.

The contact lens sealed in the contact lens package of the present disclosure may be a conventional hydrogel or and silicone hydrogel. As used herein, a "conventional hydrogel" refers to a material formed from polymerization of one or more hydrophilic monomers such as 2-hydroxyethyl methacrylate (HEMA) or vinyl alcohol, optionally in combination with other monomers, and contains no siloxane (i.e. a molecule comprising at least one Si-O group). Examples of conventional hydrogels include etafilcon A, nelfilcon A, ocufilcon B, ocufilcon D, omafilcon A, omafilcon D and polymacon. Silicone hydrogel materials are typically formed from polymerization of one or more monomers or prepolymers comprising at least one Si-O group with one or more hydrophilic monomers. Examples of silicone hydrogels include balafilcon A, comfilcon A, enfilcon A, somofilcon A, narafilcon A, narafilcon B, lotrafilcon A, stenfilcon A, and senofilcon A. In a specific example, the sealed contact lens package comprises a sterile, unworn silicone hydrogel contact lens. In a specific example, the silicone hydrogel contact lens is non-ionic, meaning it that it contains no anionic groups that bind to the cationic amine groups present in the εPLL via ionic interaction. In a further specific example, the silicone hydrogel contact lens is made from a material selected from comfilcon A or stenfilcon A. In another example, the sealed contact lens package comprises an unworn conventional hydrogel contact lens made from a material selected from ocufilcon B, ocufilcon D, or omafilcon A.

The contact lens sealed in the contact lens package of the present disclosure may be of any lens wear modality. Lens wear modality refers to the how many days and nights in a row the lens can be worn without removal. In one example, the contact lens sealed in the contact lens package of the present disclosure is a daily disposable lens. Daily disposable lenses are indicated for single use, up to about 12 or 16 hours of continuous wear, and should be discarded after the single use. In another example, the contact lens sealed in the contact lens package of the present disclosure is a daily wear lens. Daily wear lenses are worn during the waking hours, typically up to about 12 to 16 hours, and are removed before sleep. Daily wear lenses are typically stored in a contact lens case containing a contact lens care solution for cleaning and disinfecting the lens during the hours of non-use. Daily wear lenses are typically discarded after a maximum of 30 days wear. In yet another example, the contact lens is an extended wear lens. Extended wear lenses are typically worn continuously for up to 6, 14 or 30 consecutive days and nights.

The packaging solution sealed within the contact lens package of the present disclosure is a contact-lens compatible solution comprising an effective amount of εPLL. In one example, the packaging solution comprises, consists, or consists essentially, of an aqueous solution of a buffer, and/or a tonicity agent, and εPLL. In another example, the packaging solution contains additional agents such as one or more additional antimicrobial agents, and/or a comfort agent, and/or a hydrophilic polymer, and/or a surfactant and/or other additive that prevents the lens from sticking to the package. The packaging solution can have a pH in the range of about 6.8 or 7.0 up to about 7.8 or 8.0. In one example, the packaging solution comprises phosphate buffer or borate buffer. In another example, the packaging solution comprises a tonicity agent selected from sodium chloride or sorbitol in an amount to maintain osmolality in the range of about 200 to 400 mOsm/kg, and typically from about 270 mOsm/kg up to about 310 mOsm/kg. Throughout this disclosure a reference to "examples", "an example", "one example", or similar phrase, is intended to introduce a feature or features of the contact lens package, unworn contact lens, or packaging solution, as the case may be (depending on context) that can be combined with any combination of previously-described or subsequently-described examples (i.e. features), unless a particular combination of features is mutually exclusive, or if context indicates otherwise.

An effective amount of εPLL is an amount that reduces microbial contamination introduced to the lens during its removal from the contact lens package, wherein said reduced contamination is determined by a test where *Pseudomonas aeruginosa* (PA) is introduced to the lens during removal from the contact lens packaging solution and compared to a control contact lens packaged in an identical contact lens packaging solution but without said εPLL, and wherein the unworn contact less results in less than a two log kill of PA compared to the control contact lens when tested after 24 hours incubation with 10⁴ CFU PA using an *in vitro* bioactivity assay. The ability of εPLL to reduce microbial contamination introduced to a lens during its removal from a package can be demonstrated using methodology substantially as described in Example 1 or Example 2 below. Example 1 demonstrates that microbial contamination of contact lenses caused by normal, nonpathogenic skin flora can be significantly reduced by inclusion of εPLL in the contact lens packaging solution. Example 2 demonstrates that microbial contamination of contact lenses caused by *Pseudomonas aeruginosa* (PA), one of the most common pathogens implicated in microbial keratitis, can also be significantly reduced by inclusion of εPLL in the contact lens packaging solution. Advantageously, the unworn contact lens exhibits reduced contamination from PA introduced to the lens during removal from the packaging solution compared to a control contact lens in the same packaging comprising a contact lens packaging solution without εPLL but otherwise identical, as determined using a lens handling assay as described or substantially as described in Example 2. In various examples, the packaging solution comprises εPLL in amounts of at least 5 ppm, 10 ppm, 25 ppm, 50 ppm, 100 ppm, 150 ppm, 200 ppm, 250 ppm, or 500 ppm. In specific examples, the packaging solution comprises 10-50 ppm εPLL, 25-75 ppm εPLL, 50-150 ppm εPLL, or 100-500 ppm εPLL. The concentration of εPLL in a packaging solution is determined prior to its contact with the unworn contact lens.

εPLL is commercially available typically as a homopolymer ranging from about 25 to about 35 lysine (LYS) residues (CAS no. 28211-04-3). All fractions of the naturally-occurring homopolymer of εPLL may be used. Alternatively, a select fraction of the εPLL may be used (e.g. a homopolymer of 30-35 LYS residues) with the remaining fractions removed and not used in this option. As an alternative to naturally-occurring εPLL, the εPLL used in the packaging solution may be obtained from synthetic peptide methods.

The amount of εPLL included in the contact lens packaging solution results in less than a two log kill of PA compared to a control contact lens when tested after 24 hours incubation with 10⁴ CFU (colony-forming units) PA as determined using an *in vitro* bioactivity assay substantially as described in Example 4 below. Optionally, the amount of εPLL included in the contact lens packaging solution results in the contact lens of the invention having a log kill of PA compared to a control contact lens of from 0.05 to 1.95, such as from 0.10 to 1.90. The amount of εPLL included in the contact lens packaging solution may, for example, result in a log kill of PA compared to a control contact lens of from 0.05 to 1.5, such as from 0.1 to 1.0. In such examples the contact lens package remains effective against microbial contamination introduced from initial handling of the lens during its removal from its original packaging, and thus is particularly suitable for a daily disposable contact lens.

We demonstrate in Example 2 below that once the unworn contact lens is removed from the contact lens package the remaining packaging solution when covered up by the original blister foil can resist microbial contamination when left at ambient temperature for up to 16 hours. Thus, the contact lens package described herein can provide protection against microbial contamination that might arise from a non-compliant daily lens wearer who stores a worn lens overnight in its original package and remaining packaging solution for a second day of wear. Thus, the contact lens package comprises a plastic base member comprising a cavity configured to retain the contact lens and packaging solution and a flange region extending outwardly around the cavity. A removable foil is attached to the flange region to provide a sealed contact lens package. Such contact lens packages, which are commonly referred to as "blister packs", are well-known in the art (see e.g. U.S. Pat. No. 7,426,993

It will be appreciated that conventional manufacturing methods can be used to manufacture the sealed contact lens package of any of the above examples. Thus, in one aspect of the present disclosure is a method of manufacturing a contact lens package according to claim 6, including the step of placing an unworn contact lens and a contact lens packaging solution comprising εPLL in a receptacle, placing a cover on the receptacle, and sealing the cover on the receptacle. Generally, the receptacle is configured to receive a single contact lens and an amount of packaging solution sufficient to completely cover the contact lens, typically about 0.5-1.5 ml. The receptacle comprises a plastic base member comprising a cavity configured to retain the contact lens and packaging solution and a flange region extending outwardly around the cavity, and the cover comprises a removable foil attached to the flange region to provide the sealed contact lens package. The removable foil may be sealed by any conventional means such as heat sealing or gluing. The method of manufacturing the sealed contact lens package further comprises sterilizing the unworn contact lens by autoclaving the sealed contact lens package.

The details of the contact lens or lenses, components of the solution, formulations, and all of the various other details as described U.S. Patent Application No. 14/109,976, filed December 18, 2013 and entitled "Antimicrobial Ophthalmic Devices".

### EXAMPLES

The following Examples illustrate certain aspects and advantages of the present invention, which should be understood not to be limited thereby.

### Example 1: Contamination of commercial contact lenses after handling with washed hands.

Commercially available balafilcon A contact lenses in their original blister packaging were used in this lens-handling study. Five individuals washed their hands with soap and water and dried their hands using paper towels. The foil cover of each blister was opened aseptically with a gloved hand by the investigator. Each individual removed and gently rubbed the lens for approximately 5 seconds using the same hand used to turn off the faucet. Each rubbed lens was then placed into an individual sterile 2mL microcentrifuge (Eppendorf^{™}) tube with 1mL PBS-T and capped. As used herein, PBS refers phosphate buffered saline of 0.78 wt.% NaCl, 0.05 wt.% sodium phosphate monobasic, and 0.36 wt.% sodium phosphate dibasic at pH 7.5. PBS-T refers to PBS with 0.05% Polysorbate 80.

To extract any bacteria from the handled lenses, the tubes were sonicated for 3 cycles of 30 seconds each with 10 seconds vortexing in between. After the final sonication the tubes were vortexed for 10 minutes at 1000 rpm using a multi-tube vortexer. The entire volume of extract from each tube was plated onto a blood agar plate, allowed to dry in a biochemical hood, and incubated at 37°C for 48-72 hours. Bacteria were counted and reported as CFU/lens. Each bacterium with a distinct phenotype was identified using a Biotyper.

Detectable levels of contamination from common skin bacteria (Staphylococcus epidermidis and Staphylococcus warneri) were found on all five balafilcon A contact lenses. The range of contamination was from 115-6500 CFU/lens. No lens was sterile. Although the identified bacteria are not commonly found in microbial keratitis, they can cause disease in immune-compromised patients.

To determine whether εPLL in a contact lens packaging solution would be protective immediately against microbes deposited during lens removal from a blister package, the above lens-handling study was repeated using ocufilcon D, comfilcon A, and omafilcon A contact lenses that had been individually packaged in a contact lens blister package containing a packaging solution of either PBS or PBS with 500 ppm εPLL, sealed with a foil cover, and autoclaved. All three lenses demonstrated a significant reduction in bioburden when εPLL was present in the packaging solution, with the following average log kill: ocufilcon D - 2.4, comfilcon A - 1.4, and omafilcon A - 2.2.

Finally, we repeated the above lens-handling study to compare the contamination level of an ocufilcon D contact lens removed from a sealed autoclaved package containing PBS packaging solution with or without 500 ppm εPLL. We also determined the level of microbial contamination of ocufilcon D contact lenses that were removed from their sealed autoclaved packages containing PBS packaging solution. The lenses were removed from their packages aseptically using sterile forceps and placed into a well of a 24-well plate containing 1 ml of Optifree^{™} or AQuify^{™} brand contact lens solutions. Lenses exposed to ClearCare^{™} brand contact lens solutions were placed into the manufacturer-provided lens holder. After an overnight soak, the lenses (5 for each lens solution) were removed from the care solution using the above hand-washing, handling protocol and extracted as described above. As expected, the ClearCare^{™} contact lens solution, which does not contain any antimicrobial agents, did not provide protection against handling contamination. However, surprisingly, neither Optifree^{™} nor AQuify^{™} brand contact lens care solutions, both of which contain active antimicrobial agents, had any significant effect on bioburden. Only the solution containing εPLL in the blister resulted in a statistically significant reduction in bacterial count (p=0.00002). The results are shown in Table 1.

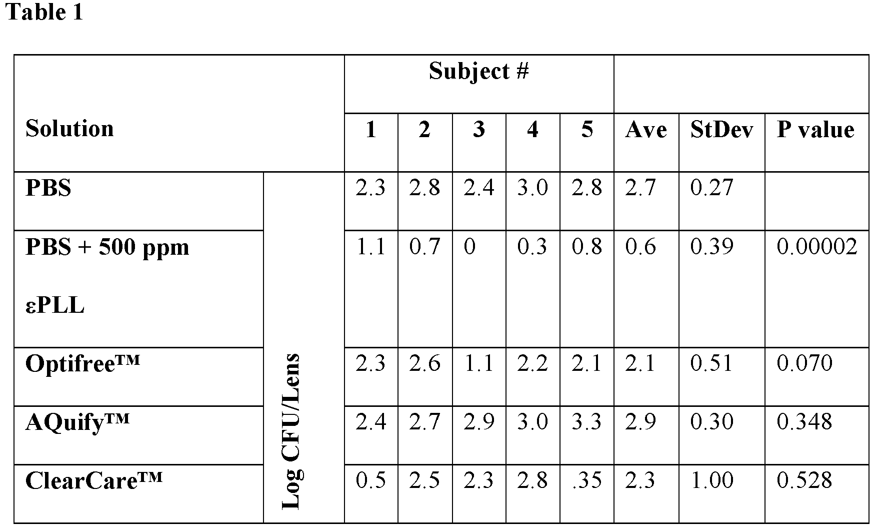

These experiments indicate that handling is a significant source of microbial contamination of conventional hydrogel and silicone hydrogel contact lenses. εPLL is protective against environmentally-derived bacteria deposited on the lens by lens handling. The carryover of εPLL from a packaging solution significantly reduced bioburden delivered by handling.

### Example 2: Lens handling assay - contamination of contact lenses from pathogens common in microbial keratitis

The bacterial adherence onto the surface of comfilcon A lenses removed from autoclaved blister packages containing a packaging solution of PBS with or without εPLL (10, 25, 100, or 500 ppm) was evaluated using a modification of methods described by Nomachi et al., Eye & Contact Lens (2013) 39:234-238. Briefly, the bacterial adherence onto the surface of comfilcon A contact lenses was evaluated using stock solutions of *Pseudomona aeruginosa* (PA) and *Staphylococcus aureus* (SA) prepared substantially as described in Example 3 below. Sterile surgical plastic gloves were put on both hands aseptically. After the seal of the comfilcon A package was opened, the thumb and index finger of a hand gloved were dipped into the bacterial suspension (approximately 10³ CFU/ml in PBS) and used to remove a lens from a package. Four lenses of each type of packaging solution were tested. Each lens was placed into a microcentrifuge tube with 1 mL PBS-T, sonicated for 1.5 minute and vortexed for 10 minutes at 1000 rpm. The entire volume of the extract was plated onto a culture dish with tryptic soy agar (TSA)and grown at 37°C for 2 days in an incubator.

Table 2 below shows the average CFU/Lens (n=4) on contact lenses removed from the different packaging solutions.

**Table 2**

| **Organism** | **0 ppm εPLL** | **10 ppm εPLL** | **25 ppm εPLL** | **100 ppm εPLL** | **500 ppm εPLL** |
|---|---|---|---|---|---|
| **PA** | 54 | 42 | 16 | 1 | 0 |
| **SA** | 43 | 37 | 35 | 21 | 7 |

After removal of the contact lenses by finger and thumb contaminated with PA, as described above, the blister package and remaining packaging solution were left covered with the original blister foil at ambient temperature. After 16 hours, the entire volume of remaining packaging solution (~ 1mL) was plated onto a culture dish with TSA and left in an incubator at 37°C for 2 days to grow. The packaging solution without any added ePL had an average of 331 CFU/Lens. None of the ePL-containing packaging solutions exhibited microbial growth, i.e. all had 0 CFU/Lens.

### Example 3: Preparation of bacterial suspensions

Cultures were prepared from growing a single colony of each of the bacterial species shown in Table 3 below in 50 mL. trypticase soy broth (TSB) overnight at 37 °C on a rotary shaker. 1 mL of each culture was centrifuged, and the bacterial pellet resuspended in 1.0 mL of the diluent shown in Table 3. For each bacterial species, a suspension of approximately 10⁸ CFU/mL was prepared by diluting the bacterial suspension to achieve the optical density indicated in Table 3. Each suspension is further diluted for use in the handling assay described in Example 2 or the *in vitro* bioactivity assay described in Example 4.

**Table 3**

| **Species** | **Strain** | **OD₆₆₀** | **Media Diluent** |
|---|---|---|---|
| PA | ATCC 99027 | ∼0.1 | 0.01 % TSB in PBS-T |
| SA | ATCC 13880 | ∼0.3 | 10.0 % TSB in PBS-T |

### Example 4: In Vitro Bioactivity Assay

A contact lens is removed from its packaging and rinsed in 2.5 ml sterile PBS for a few seconds (e.g. 4 seconds) to remove residual packaging solution. The rinsed lens is then transferred to an individual well of a 24-well plate containing 1.0 mL of 10⁴ CFU PA. The plate is incubated at 37°C with gentle shaking for 24 hours. The lens is removed from its well and transferred to a well of a 12-well plate containing 2.5 mL of sterile PBS. The plate is gently swirled for about 30 seconds. This step is repeated once for each lens.

Each washed lens is placed in a microcentrifuge tube containing 1 mL of Dey-Engley (DE) neutralizing broth and adhered bacteria are removed by a combination of sonication for about 2 minutes and vortexing for about 10 minutes. Serial dilutions are made for each recovered cell suspension using DE neutralizing broth and suitable dilutions are plated onto TSA. Plates are incubated overnight at 37°C and CFUs are counted.

The CFU for each plate is multiplied by the dilution factor (DF) as well as the plating dilution factor (PDF). The total CFUs recovered for a given sample are then converted to the log 10. To calculate the log kill of a contact lens packaged in a packaging solution comprising εPLL (i.e. the "test lens"), the log of CFU/lens of the test lens is subtracted from that of a control lens, which is identical to the test lens and packaged in an identical package and contact lens packaging solution except that the packaging solution lacks the εPLL. For example, if the mean log 10 value of an antimicrobial lens is 1.05 and the mean log 10 value of an otherwise identical control lens lacking the active antimicrobial agent is 5.52, the log kill is 5.52-1.05 = 4.47.

Although the disclosure herein refers to certain illustrated examples, it is to be understood that these examples are presented by way of example and not by way of limitation.

The present invention provides a sealed, autoclaved contact lens package comprising a sterile, unworn contact lens and a contact lens packaging solution comprising epsilon polylysine (εPLL), wherein the unworn contact lens exhibits reduced contamination from at least one microbe, wherein said reduced contamination is determined by a test where *Pseudomonas aeruginosa* (PA) introduced to the lens during removal from the contact lens packaging solution compared to a control contact lens packaged in an identical contact lens packaging solution but without said εPLL, wherein, in the test, the unworn contact less results in less than a two log kill of PA compared to the control contact lens when tested after 24 hours incubation with 10⁴ CFU PA using an *in vitro* bioactivity assay, wherein the contact lens is a conventional hydrogel contact lens or silicone hydrogel contact lens, and wherein the sealed contact lens package comprises a plastic base member that comprises a cavity configured to retain the contact lens and the packaging solution, and a flange region extending outwardly around the cavity, and a removable foil attached to the flange region to provide the sealed contact lens package. Advantageously, in the test, the unworn contact less results in less than a one log kill of PA (but more than a zero log kill) compared to the control contact lens when tested after 24 hours incubation with 10⁴ CFU PA using an *in vitro* bioactivity assay.

Optionally, the contact lens is a silicone hydrogel contact lens. The contact lens may be a non-ionic contact lens or an ionic contact lens. The contact lens may be a daily disposable contact lens or a daily wear contact lens.

The packaging solution optionally comprises between 5 ppm and 500 ppm εPLL,such as between 5 ppm and 50 ppm εPLL.

The at least one microbe is optionally *Pseudomonas aeruginosa, Staphylococcus aureus* or at least one microbe is a microbe found in microbial keratitis.

The invention further provides a method of manufacturing the sealed contact lens package, said method comprising:
(a) placing an unworn contact lens and a contact lens packaging solution comprising εPLL in a receptacle configured to receive a contact lens and sealing the receptacle with a cover to provide a sealed contact lens package, wherein the receptacle comprises plastic base member that comprises the cavity configured to retain the contact lens and packaging solution, and the flange region extending outwardly around the cavity;
(b) sealing the receptacle with a cover that comprises the removable foil attached to the flange region to provide the sealed contact lens package; and
(c) sterilizing the unworn contact lens by autoclaving the sealed contact lens package,
wherein the unworn contact lens exhibits reduced contamination from *Pseudomonas aeruginosa* (PA) introduced to the lens during removal from the contact lens packaging solution compared to a control contact lens packaged in a substantially identical contact lens packaging solution without εPLL, and wherein the unworn contact less results in less than a two log kill of PA compared to the control contact lens when tested after 24 hours incubation with 10⁴ CFU PA using an *in vitro* bioactivity assay.

## Claims

1. A sealed, autoclaved contact lens package comprising a sterile, unworn contact lens and a contact lens packaging solution comprising epsilon polylysine (εPLL),
wherein the unworn contact lens exhibits reduced contamination from at least one microbe, wherein said reduced contamination is determined by a test where *Pseudomonas aeruginosa* (PA) is introduced to the lens during removal from the contact lens packaging solution and compared to a control contact lens packaged in an identical contact lens packaging solution but without said εPLL, and wherein the unworn contact less results in less than a two log kill of PA compared to the control contact lens when tested after 24 hours incubation with 10⁴ CFU PA using an *in vitro* bioactivity assay,
wherein the contact lens is a conventional hydrogel contact lens or silicone hydrogel contact lens,
and wherein the sealed contact lens package comprises a plastic base member that comprises a cavity configured to retain the contact lens and the packaging solution, and a flange region extending outwardly around the cavity, and a removable foil attached to the flange region to provide the sealed contact lens package.

2. The package of any claim 1, wherein the contact lens is a non-ionic contact lens.

3. The package of any preceding claim, wherein the contact lens is a daily wear contact lens.

4. The package of any preceding claim, wherein the packaging solution comprises between 5 ppm and 500 ppm εPLL.

5. The package of any preceding claim, wherein the at least one microbe is *Pseudomonas aeruginosa, Staphylococcus aureus* or a microbe found in microbial keratitis.

6. A method of manufacturing the sealed contact lens package of any one of claims 1 to 5, said method comprising:
placing the unworn contact lens and the contact lens packaging solution comprising epsilon polylysine (εPLL) in a receptacle configured to receive a contact lens that comprises the plastic base member that comprises the cavity configured to retain the contact lens and packaging solution, and the flange region extending outwardly around the cavity;
sealing the receptacle with a cover that comprises the removable foil attached to the flange region to provide the sealed contact lens package; and
sterilizing the unworn contact lens by autoclaving the sealed contact lens package.

## Patentansprüche

1. Versiegelte autoklavierte Kontaktlinsenverpackung mit einer sterilen ungetragenen Kontaktlinse und einer Kontaktlinsenverpackungslösung mit Epsilon-Polylysin (εPLL),
wobei die ungetragene Kontaktlinse eine reduzierte Kontamination bezüglich mindestens eines Mikroorganismus zeigt, wobei die reduzierte Kontamination durch eine Prüfung ermittelt ist, in der *Pseudomonas aeruginosa* (PA) in die Linse während ihres Entfernens aus der Kontaktlinsenverpackungslösung eingeführt und mit einer Kontrollkontaktlinse, die in einer identischen Kontaktlinsenverpackungslösung aber ohne das εPLL verpackt ist, verglichen wird, und wobei die ungetragene Kontaktlinse zu weniger als Zwei-Log-Vernichtung an PA im Vergleich zu der Kontrollkontaktlinse führt, wenn nach 24 Stunden Inkubation mit 10⁴ CFU PA unter Anwendung einer In-vitro-Bioaktivitätsuntersuchung geprüft wird,
wobei die Kontaktlinse eine konventionelle Hydrogelkontaktlinse oder eine Silikonhydrogelkontaktlinse ist,
und wobei die versiegelte Kontaktlinsenverpackung ein Kunststoffgrundelement aufweist, das eine Aussparung, die dazu ausgebildet ist, die Kontaktlinse und die Verpackungslösung aufzunehmen, und einen Flanschbereich aufweist, der sich um die Aussparung herum nach außen erstreckt, und ferner eine abnehmbare Folie aufweist, die derart an dem Flanschbereich angebracht ist, dass sie die versiegelte Kontaktlinsenverpackung bereitstellt.

2. Verpackung nach Anspruch 1, wobei die Kontaktlinse eine nicht-ionische Kontaktlinse ist.

3. Verpackung nach einem vorhergehenden Anspruch, wobei die Kontaktlinse eine Kontaktlinse zur täglichen Nutzung ist.

4. Verpackung nach einem vorhergehenden Anspruch, wobei die Verpackungslösung zwischen 5 ppm und 500 ppm an εPLL enthält.

5. Verpackung nach einem vorhergehenden Anspruch, wobei der mindestens eine Mikroorganismus *Pseudomonas aeruginosa, Staphylococcus aureus* oder ein Mikroorganismus ist, der in mikrobieller Keratitis vorhanden ist.

6. Verfahren zur Herstellung der versiegelten Kontaktlinsenverpackung nach einem der Ansprüche 1 bis 5, wobei das Verfahren umfasst:
Anordnen der ungetragenen Kontaktlinse und der Kontaktlinsenverpackungslösung, die Epsilon-Polylysin (εPLL) aufweist, in einer Aufnahme, die dazu ausgebildet ist, eine Kontaktlinse aufzunehmen, die das Kunststoffgrundelement aufweist, das die Aussparung bildet, die dazu ausgebildet ist, die Kontaktlinse und die Verpackungslösung aufzunehmen, wobei der Flanschbereich sich um die Aussparung herum nach außen erstreckt;
Versiegeln der Aufnahme mit einer Abdeckung, die die abnehmbare Folie, die an dem Flanschbereich angebracht ist, aufweist, um die versiegelte Kontaktlinsenverpackung bereitzustellen; und
Sterilisieren der ungetragenen Kontaktlinse durch Autoklavieren der versiegelten Kontaktlinsenverpackung.

## Revendications

1. Emballage pour lentilles de contact, scellé et passé à l'autoclave, comprenant une lentille de contact stérile non portée et une solution d'emballage pour lentilles de contact comprenant une epsilon polylysine (εPLL),
dans lequel la lentille de contact non portée présente une contamination réduite par au moins un microbe, dans lequel ladite contamination réduite est déterminée par un test dans lequel *Pseudomonas aeruginosa* (PA) est introduit dans la lentille lors du retrait de la solution d'emballage pour lentilles de contact et par rapport à une lentille de contact témoin emballée dans une solution d'emballage pour lentilles de contact identique mais sans ladite εPLL, et dans lequel la lentille de contact non portée donne une destruction de PA de moins de deux log par rapport à la lentille de contact témoin lors d'un test après 24 heures d'incubation avec 10⁴ UFC de PA en utilisant un test de bioactivité *in vitro,*
dans lequel la lentille de contact est une lentille de contact en hydrogel classique ou une lentille de contact en silicone hydrogel,
et dans lequel l'emballage pour lentilles de contact scellé comprend un élément de base en plastique qui comprend une cavité configurée pour retenir la lentille de contact et la solution d'emballage, et une région de rebord s'étendant vers l'extérieur autour de la cavité, et une feuille amovible fixée à la région de rebord pour fournir l'emballage pour lentilles de contact scellé.

2. Emballage selon la revendication 1, dans lequel la lentille de contact est une lentille de contact non ionique.

3. Emballage selon une quelconque revendication précédente, dans lequel la lentille de contact est une lentille de contact pour port quotidien.

4. Emballage selon une quelconque revendication précédente, dans lequel la solution d'emballage comprend entre 5 ppm et 500 ppm d'εPLL.

5. Emballage selon une quelconque revendication précédente, dans lequel l'au moins un microbe est *Pseudomonas aeruginosa, Staphylococcus aureus* ou un microbe trouvé lors d'une kératite microbienne.

6. Procédé de fabrication de l'emballage pour lentilles de contact scellé selon l'une quelconque des revendications 1 à 5, ledit procédé comprenant :
le placement de la lentille de contact non portée et de la solution d'emballage pour lentilles de contact comprenant une epsilon polylysine (εPLL) dans un réceptacle configuré pour recevoir une lentille de contact qui comprend l'élément de base en plastique qui comprend la cavité configurée pour retenir la lentille de contact et la solution d'emballage, et la région de rebord s'étendant vers l'extérieur autour de la cavité ;
le scellement du réceptacle avec un couvercle qui comprend la feuille amovible fixée à la région de rebord pour fournir l'emballage pour lentilles de contact scellé ; et
la stérilisation de la lentille de contact non portée par le passage à l'autoclave de l'emballage pour lentilles de contact scellé.
